(19) Europäisches Patentamt
European Patent Office
Office européen des brevets

(11) Veröffentlichungsnummer : **0 007 505**
**B1**

# (12) EUROPÄISCHE PATENTSCHRIFT

(45) Veröffentlichungstag der Patentschrift : **30.09.81**

(51) Int. Cl.³ : **C 07 D249/08**, C 07 D233/60, A 01 N 43/50, A 01 N 43/64

(21) Anmeldenummer : **79102342.7**

(22) Anmeldetag : **09.07.79**

(54) **Alpha-Azolyl-beta-hydroxy-ketone, Verfahren zu ihrer Herstellung und ihre Verwendung als Fungizide.**

(30) Priorität : **21.07.78 DE 2832233**

(43) Veröffentlichungstag der Anmeldung : **06.02.80 (Patentblatt 80/03)**

(45) Bekanntmachung des Hinweises auf die Patenterteilung . **30.09.81 Patentblatt 81/39**

(84) Benannte Vertragsstaaten : **AT BE CH DE FR GB IT NL SE**

(56) Entgegenhaltungen :
**DE - A - 2 713 022**
**DE - A - 2 734 426**
**DE - A - 2 737 489**

(73) Patentinhaber : **BAYER AG**
**Zentralbereich Patente, Marken und Lizenzen**
**D-5090 Leverkusen 1, Bayerwerk (DE)**

(72) Erfinder : **Kranz, Eckart, Dr.**
**Am Acker 9**
**D-5600 Wuppertal 1 (DE)**
Erfinder : **Krämer, Wolfgang, Dr.**
**Am Eckbusch 39/162**
**D-5600 Wuppertal 1 (DE)**
Erfinder : **Büchel, Karl Heinz, Dr. Prof.**
**Bergerheide 62**
**D-5600 Wuppertal 1 (DE)**
Erfinder : **Brandes, Wilhelm, Dr.**
**Eichendorffstrasse 3**
**D-5653 Leichlingen 1 (DE)**
Erfinder : **Frohberger, Paul-Ernst, Dr.**
**Willi-Baumeister-Strasse 5**
**D-5090 Leverkusen 1 (DE)**

**EP 0 007 505 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Alpha-Azolyl-beta-hydroxy-ketone, Verfahren zu ihrer Herstellung und ihre Verwendung als Fungizide

Die vorliegende Erfindung betrifft neue α-Azolyl-β-hydroxy-ketone, ein Verfahren zu ihrer Herstellung und ihre Verwendung als Fungizide.

Es ist bereits bekannt geworden, daß substituierte Phenoxy-triazolyl-keto- bzw. -hydroxy-Derivate im allgemeinen sehr gute fungizide Eigenschaften aufweisen (vergleiche DE-AS 2 201 063 [Le A 14 118] bzw. DE-OS 2 324 010 [Le A 14 971]. Die Wirkung ist jedoch, insbesondere bei niedrigen Aufwandmengen und -konzentrationen, in einigen speziellen Anwendungsbereichen nicht immer voll befriedigend.

Es wurden neue -Azolyl-β-hydroxy-ketone der allgemeinen Formel

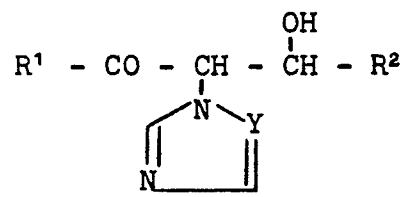

(I)

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, Alkylsulfonyloxy mit 1 bis 4 Kohlenstoffatomen und durch Phensulfonyloxy, wobei letzteres durch Alkyl mit 1 bis 2 Kohlenstoffatomen substituiert sein kann, ferner für Phenyl steht, welches gegebenenfalls substituiert sein kann durch Halogen, Alkyl mit bis zu 4 Kohlenstoffatomen, Phenyl und Phenoxy, wobei die beiden letztgenannten Reste gegebenenfalls halogensubstituiert sein können,

$R^2$ für die Gruppierung $-CX^1X^2R^3$ oder Alkoxycarbonyl mit 1 bis 4 Kohlenstoffatomen im Alkoxyteil steht, wobei

$R^3$ für Halogen und Halogenalkyl mit 1 bis 4 Kohlenstoffatomen und 1 bis 3 Halogenatomen steht,

$X^1$ und $X^2$ gleich oder verschieden sind und für Wasserstoff oder Halogen stehen und

Y für ein Stickstoffatom oder die CH-Gruppe steht,

und deren physiologisch verträglichen Säureadditions-Salze und Metallsalz-Komplexe gefunden. Sie weisen starke fungizide, insbesondere systemische fungizide Eigenschaften auf.

Die Verbindungen der Formel (I) besitzen zwei asymmetrische Kohlenstoffatome ; sie können deshalb in der erythro- wie in der threo-Form vorliegen. In beiden Fällen liegen sie vorwiegend als Racemate vor.

Man erhält die α-Azolyl-β-hydroxy-ketone der Formel (I), wenn man α-Azolyl-ketone der Formel

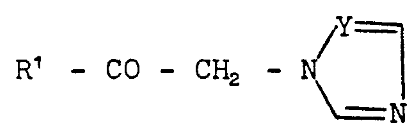

(II)

in welcher

$R^1$ und Y die oben angegebene Bedeutung haben, mit Aldehyden der Formel

$R^2-CHO$ (III)

in welcher

$R^2$ die oben angegebene Bedeutung hat, in Gegenwart eines Lösungsmittels und in Gegenwart eines Katalysators umsetzt.

In manchen Fällen können die Aldehyde der Formel (III) auch in Form ihrer Hydrate oder Halbacetale eingesetzt werden.

Weiterhin können die erfindungsgemäß erhältlichen α-Azolyl-β-hydroxy-ketone der Formel (I) durch Umsetzen mit Säuren in die Salze überführt werden, bzw. können durch Reaktion mit Metallsalzen die entsprechenden Metallsalz-Komplexe erhalten werden.

Ueberraschenderweise zeigen die erfindungsgemäßen α-Azolyl-β-hydroxy-ketone eine erheblich höhere fungizide Wirksamkeit, insbesondere bei systemischer Anwendung gegen Schorf- und Mehltauarten, als die aus dem Stand der Technik bekannten substituierten Phenoxy-triazolyl-keto- bzw. -hydroxy-Derivate, welche chemisch und wirkungsmäßig naheliegende Verbindungen sind. Die erfindungsgemäßen Stoffe stellen somit eine Bereicherung der Technik dar.

Ganz besonders bevorzugt sind diejenigen Verbindungen der Formel (I), in denen $R^1$ für tert.-Butyl, sek.-Butyl, Isopropyl, Chlor-tert.-butyl, Brom-tert.-butyl, Fluor-tert.-butyl, 1,3-Dichlor-2-methyl-prop-2-yl, Methylsulfonyloxy-tert.-butyl, Phenylsulfonyloxy-tert.-butyl, Acetoxy-tert.-butyl und Ethylcarbonyloxy-tert.-butyl steht ; ferner für Phenyl, Chlorphenyl, Dichlorphenyl, Chlormethyl-phenyl, Bromphenyl, Biphenylyl, Chlorbiphenylyl, Phenoxyphenyl oder Chlorphenoxyphenyl steht ; $R^2$ für Trichlormethyl,

Dichlorfluormethyl, Dichlormethyl, Chlormethyl, 1,1,2-Tribromethyl, 1,1,2-Trichlor-prop-1-yl, Methoxycarbonyl oder Ethoxycarbonyl steht und *Y* für ein Stickstoffatom oder die CH-Gruppe steht.

Im einzelnen seien außer den bei den Herstellungsbeispielen angegebenen Verbindungen die folgenden Verbindungen der allgemeinen Formel (I) genannt :

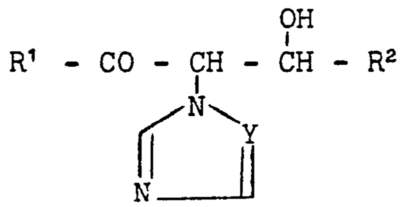

| $R^1$ | $R^2$ | Y |
|---|---|---|
| 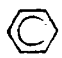 | $-CO-O-C_2H_5$ | N |
| 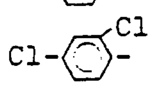 | $-CO-O-C_2H_5$ | N |
| 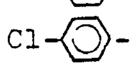 | $-CO-O-C_2H_5$ | N |
| 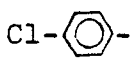 | $-CCl_2-CHCl-CH_3$ | N |
| 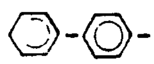 | $-CCl_2-CHCl-CH_3$ | N |
| 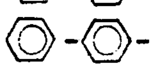 | $-CO-O-C_2H_5$ | N |
| 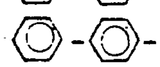 | $-CCl_3$ | N |
| 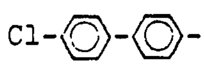 | $-CCl_3$ | N |
| 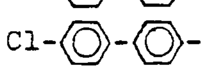 | $-CO-O-C_2H_5$ | N |
| 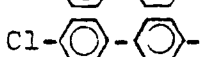 | $-CCl_2-CHCl-CH_3$ | N |
| 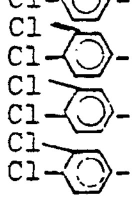 | $-CCl_3$ | N |
| | $-CO-O-C_2H_5$ | N |
| | $-CCl_2-CHCl-CH_3$ | N |
| 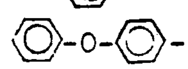 | $-CCl_3$ | N |
| 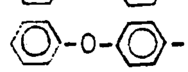 | $-CO-O-C_2H_5$ | N |
| 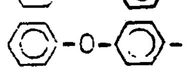 | $-CCl_2-CHCl-CH_3$ | N |
| 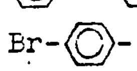 | $-CCl_3$ | N |
| 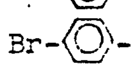 | $-CO-O-C_2H_5$ | N |
| 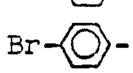 | $-CCl_2-CHCl-CH_3$ | N |
| 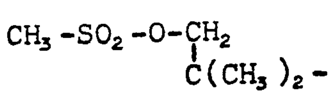 | $-CCl_3$ | N |
| 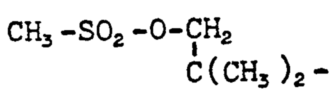 | $-CO-O-C_2H_5$ | N |

| $R^1$ | $R^2$ | Y |
|---|---|---|
| $CH_3-SO_2-O-CH_2-C(CH_3)_2-$ | $-CCl_2-CHCl-CH_3$ | N |
| $CH_3-CO-O-CH_2-C(CH_3)_2-$ | $-CCl_3$ | N |
| $CH_3-CO-O-CH_2-C(CH_3)_2-$ | $-CO-O-C_2H_5$ | N |
| $CH_3-CO-O-CH_2-C(CH_3)_2-$ | $-CCl_2-CHCl-CH_3$ | N |
| $Br-CH_2-C(CH_3)_2-$ | $-CCl_2-CHCl-CH_3$ | N |
| $Br-CH_2-C(CH_3)_2-$ | $-CO-O-C_2H_5$ | N |
| 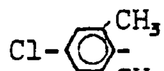 | $-CCl_3$ | N |
| 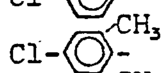 | $-CO-O-C_2H_5$ | N |
| 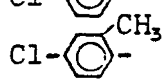 | $-CCl_2-CHCl-CH_3$ | N |
| $C(CH_3)_3$ | $-CO-O-C_2H_5$ | CH |
| 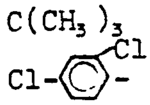 | $-CO-O-C_2H_5$ | CH |
| 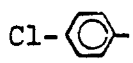 | $-CO-O-C_2H_5$ | CH |
| 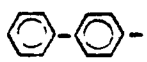 | $-CO-O-C_2H_5$ | CH |
| 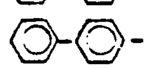 | $-CCl_2-CHCl-CH_3$ | CH |
| 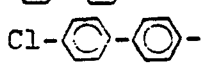 | $-CCl_3$ | CH |
| 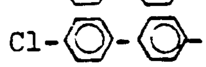 | $-CO-O-C_2H_5$ | CH |
| 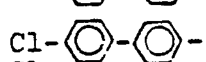 | $-CCl_2-CHCl-CH_3$ | CH |
| 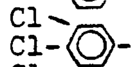 | $-CCl_3$ | CH |
| 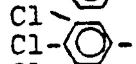 | $-CO-O-C_2H_5$ | CH |
| 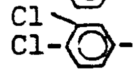 | $-CCl_2-CHCl-CH_3$ | CH |
| 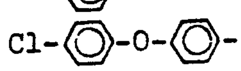 | $-CCl_3$ | CH |
| 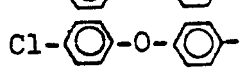 | $-CCl_2-CHCl-CH_3$ | CH |
| 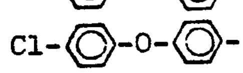 | $-CO-O-C_2H_5$ | CH |
| 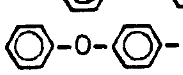 | $-CCl_3$ | CH |
| 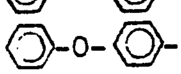 | $-CO-O-C_2H_5$ | CH |
| 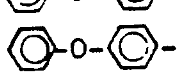 | $-CCl_2-CHCl-CH_3$ | CH |
| 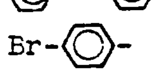 | $-CCl_3$ | CH |
| 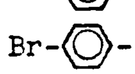 | $-CO-O-C_2H_5$ | CH |

| $R^1$ | $R^2$ | Y |
|---|---|---|
| 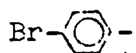 | $-CCl_2-CHCl-CH_3$ | CH |
| 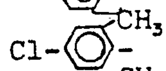 | $-CCl_3$ | CH |
| 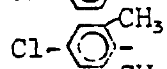 | $-CO-O-C_2H_5$ | CH |
| 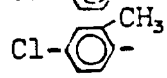 | $-CCl_2-CHCl-CH_3$ | CH |
| $Cl-CH_2-C(CH_3)_3-$ | $-CCl_3$ | CH |
| $Cl-CH_2-C(CH_3)_2-$ | $-CO-O-C_2H_5$ | CH |
| $Cl-CH_2-C(CH_3)_2-$ | $-CCl_2-CHCl-CH_3$ | CH |
| $CH_3-CO-O-CH_2-C(CH_3)_2-$ | $-CCl_3$ | CH |
| $CH_3-CO-O-CH_2-C(CH_3)_2-$ | $-CO-O-C_2H_5$ | CH |
| $CH_3-CO-O-CH_2-C(CH_3)_2-$ | $-CCl_2-CHCl-CH_3$ | CH |
| $CH_3-SO_2-O-CH_2-C(CH_3)_2-$ | $-CCl_3$ | CH |
| $CH_3-SO_2-O-CH_2-C(CH_3)_2-$ | $-CO-O-C_2H_5$ | CH |
| $CH_3-SO_2-O-CH_2-C(CH_3)_2-$ | $-CCl_2-CHCl-CH_3$ | CH |
| $Br-CH_2-C(CH_3)_2-$ | $-CO-O-C_2H_5$ | CH |
| $Br-CH_2-C(CH_3)_2-$ | $-CCl_2-CHCl-CH_3$ | CH |
| $FCH_2-C(CH_3)_2-$ | $-CCl_3$ | CH |
| $FCH_2-C(CH_3)_2-$ | $-CO-O-C_2H_5$ | CH |
| $FCH_2-C(CH_3)_2-$ | $-CCl_2-CHCl-CH_3$ | CH |

Verwendet man beispielsweise 3,3-Dimethyl-1-(1,2,4-triazol-1-yl)-butan-2-on und Chloral als Ausgangsstoffe, so kann der Reaktionsablauf durch das folgende Formelschema wiedergegeben werden :

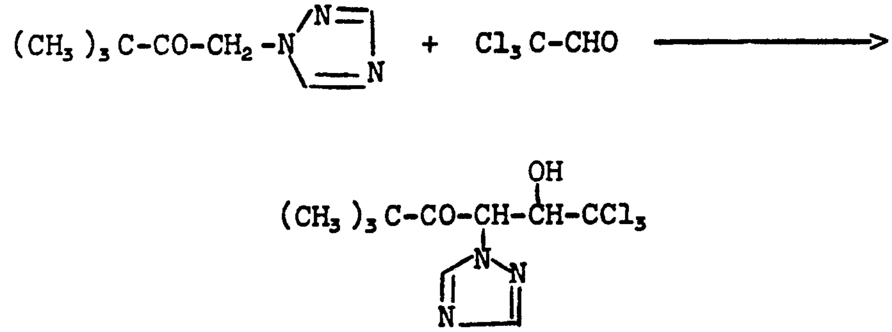

Die für das erfindungsgemäße Verfahren als Ausgangsstoffe zu verwendenden α-Azolyl-ketone sind durch die Formel (II) allgemein definiert. In dieser Formel stehen *R¹* und *Y* vorzugsweise für die Reste, die bei den Verbindungen der Formel (I) bereits vorzugsweise genannt wurden.

Die α-Azolyl-ketone der Formel (II) sind teilweise bekannt (vergleiche DE-OS 2 063 857 und DE-OS 2 431 407 [Le A 15 735]). Die noch nicht bekannten können nach den dort beschriebenen Verfahren erhalten werden, indem man z.B.

*a)* Halogenketone der Formel

$R^1$ - CO - $CH_2$ - Hal (IV)

in welcher

$R^1$ die oben angegebene Bedeutung hat und

Hal für Chlor und Brom steht,

mit 1,2,4-Triazol oder Imidazol in Gegenwart eines inerten organischen Lösungsmittels, wie beispielsweise Aceton, und in Gegenwart eines Säurebindemittels, wie beispielsweise Natriumcarbonat, vorzugsweise in der Siedehitze umsetzt (vgl. auch die Herstellungsbeispiele), oder

*b)* Hydroxyketone der Formel

$R^1$ - CO - $CH_2$ - OH (V)

in welcher

$R^1$ die oben angegebene Bedeutung hat,

mit Thionyl-bis-azolen der Formel

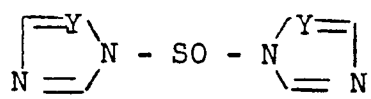

(VI)

in welcher

Y die oben angegebene Bedeutung hat,

in Gegenwart eines polaren organischen Lösungsmittels, wie beispielsweise Acetonitril, vorzugsweise in der Siedehitze umsetzt.

Als Beispiele für die Ausgangsstoffe der Formel (II) seien genannt (Azolyl steht für 1,2,4-Triazol-1-yl oder Imidazol-1-yl) :

1-Azolyl-3,3-dimethyl-butan-2-on
1-Azolyl-4-chlor-3,3-dimethyl-butan-2-on
1-Azolyl-4-brom-3,3-dimethyl-butan-2-on
1-Azolyl-4-fluor-3,3-dimethyl-butan-2-on
1-Azolyl-4-acetoxy-3,3-dimethyl-butan-2-on
1-Azolyl-4-ethylcarbonyloxy-3,3-dimethyl-butan-2-on
1-Azolyl-4-methylsulfonyloxy-3,3-dimethyl-butan-2-on
1-Azolyl-4-phenylsulfonyloxy-3,3-dimethyl-butan-2-on
ω-Azolyl-acetophenon
ω-Azolyl-4-chloracetophenon
ω-Azolyl-4-bromacetophenon
ω-Azolyl-2,4-dichloracetophenon
ω-Azolyl-4-chlor-2-methylacetophenon
ω-Azolyl-3,4-dichloracetopnenon
ω-Azolyl-4-phenylacetophenon
ω-Azolyl-4-phenoxyacetophenon
ω-Azolyl-4-(4′-chlorphenyl)-acetophenon
ω-Azolyl-4-(4′-chlorphenoxy)-acetophenon

Die außerdem für die erfindungsgemäße Umsetzung als Ausgangsstoffe zu verwendenden Aldehyde sind durch die Formel (III) allgemein definiert. In dieser Formel steht $R^2$ vorzugsweise für die Reste, die bei den Verbindungen der Formel (I) bereits vorzugsweise genannt wurden.

Die Aldehyde der Formel (III) sind allgemein bekannte Verbindungen der organischen Chemie. Als Beispiele seien genannt :

Chloral, Dichlorfluoracetaldehyd, 2,2,3-Trichlorbutyraldehyd, Glyoxylsäuremethylester, Glyoxylsäureethylester, Dichloracetaldehyd, Chloracetaldehyd, 2,2,3-Tribrompropionaldehyd.

Als Lösungsmittel kommen für die erfindungsgemäße Umsetzung vorzugsweise inerte organische Lösungsmittel infrage. Hierzu gehören vorzugsweise Alkohole, wie Methanol und Ethanol, sowie deren Mischungen mit Wasser ; Ether, wie Tetrahydrofuran und Dioxan ; Nitrile, wie Acetonitril und Propionitril ; halogenierte aliphatische und aromatische Kohlenwasserstoffe, wie Methylenchlorid, Tetrachlorkohlenstoff, Chloroform, Chlorbenzol und Dichlorbenzol ; sowie Eisessig.

Die erfindungsgemäße Umsetzung wird in Gegenwart eines Katalysators vorgenommen. Man kann alle üblicherweise verwendbaren sauren und insbesondere basischen Katalysatoren einsetzen. Hierzu gehören vorzugsweise Lewis-Säuren, wie z.B. Eisen-(III)-chlorid, Eisen-(III)-bromid, Bortrifluorid, Bortrichlorid, Zinntetrachlorid oder Titantetrachlorid ; Alkali- und Erdalkalihydroxide, wie Kaliumhydroxid, Natriumhydroxid, Calciumhydroxid oder Bariumhydroxid ; Alkalisalze, wie Kaliumcarbonat, Natriumcarbonat, Kaliumcyanid, sekundäres Natriumphosphat, Natriumacetat oder Natriumsulfit ; sowie Alkoholate, wie Natrium- oder Kaliummethylat.

Die Reaktionstemperaturen können bei der Durchführung des erfindungsgemäßen Verfahrens in einem größeren Bereich variiert werden. Im allgemeinen arbeitet man zwischen 0 und 100 °C,

vorzugsweise bei Raumtemperatur bzw. dem Siedepunkt des jeweiligen Lösungsmittels.

Bei der Durchführung des erfindungsgemäßen Verfahrens arbeitet man vorzugsweise in molaren Mengen, wobei katalytische bis molare Mengen an Katalysator eingesetzt werden. Die Isolierung der Verbindungen der Formel (I) erfolgt in üblicher Weise (vergleiche auch die Herstellungsbeispiele).

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV.-Haupt- und der I. und II. sowie IV. bis VIII. Nebengruppe infrage, wobei Kupfer, Zink, Mangen, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Aethanol, und Hinzufügen zur Verbindung der Formel (I). Man kann Metallsalzkomplexe in bekannter Weise, z.B. durch Abfiltrieren, isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen ; so zur Bekämpfung von Erysiphe-Arten, wie z.B. gegen den Erreger des Gurkenmehltaus (Erysiphe cichoreacearum) oder des Gersten- bzw. des Getreidemehltaus (Erysiphe graminis) ; sowie zur Bekämpfung solcher Pilze, die Schorferkrankungen hervorrufen ; so zur Bekämpfung von Venturia-Arten, wie z.B. gegen den Erreger des Apfelschorfs (Fusicladium dendriticum). Besonders hervorzuheben ist, daß die erfindungsgemäßen Wirkstoffe nicht nur eine protektive Wirkung entfalten, sondern insbesondere auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie

Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Mit nachfolgenden Beispielen werden einige Verwendungsmöglichkeiten eingehender dargestellt :

## Beispiel A

Erysiphe-Test (Gurken)/Systemisch

| | | |
|---|---|---|
| Lösungsmittel | : | 4,7 Gewichtsteile Aceton |
| Emulgator | : | 0,3 Gewichtsteile Alkyl-aryl-polyglykoläther |
| Wasser | : | 95,0 Gewichtsteile |

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Gurkenpflanzen werden im 1- bis 2-Blattstadium innerhalb einer Woche dreimal mit 10 ccm Gießflüssigkeit in der angegebenen Wirkstoffkonzentration, bezogen auf 100 ccm Erde, gegossen.

Die so behandelten Pflanzen werden nach der Behandlung mit Konidien des Pilzes Erysiphe cichoreacearum inokuliert. Anschließend werden die Pflanzen bei 23 bis 24 °C und einer relativen Luftfeuchtigkeit von 70 % im Gewächshaus aufgestellt.

Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen aus dem Stand der Technik bekannten Verbindungen überlegen ist :

Verbindungen gemäß Herstellungsbeispielen : 14 und 1.

## Beispiel B

Fusicladium-Test (Apfel)/Systemisch

| | | |
|---|---|---|
| Lösungsmittel | : | 4,7 Gewichtsteile Aceton |
| Emulgator | : | 0,3 Gewichtsteile Alkyl-aryl-polyglykoläther |
| Wasser | : | 95,0 Gewichtsteile |

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Apfelsämlinge werden im 3- bis 4-Blattstadium innerhalb einer Woche einmal mit 10 ccm der Gießflüssigkeit in der angegebenen Wirkstoffkonzentration, bezogen auf 100 ccm Erde, gegossen.

Die so behandelten Pflanzen werden nach der Behandlung mit einer wässrigen Konidiensuspension von Fusicladium dentriticum inokuliert und 18 Stunden lang in einer Feuchtkammer bei 18 bis 20 °C und 100 % relativer Luftfeuchtigkeit inkubiert. Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist : Verbindung gemäß Herstellungsbeispiel : 1.

## Beispiel C

Sproßbehandlungs-Test/Getreidemehltau/protektiv (blattzerstörende Mykose).

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykol-ether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrad werden ermittelt. Bei diesem Test zeigen z.B. folgende Verbindungen eine sehr gute Wirkung, die derjenigen der aus dem Stand der Technik bekannten Verbindungen überlegen ist :

Verbindungen gemäß Herstellungsbeispielen 14 und 1.

## Beispiel D

Gerstenmehltau-Test (Erysiphe graminis var. hordei)/systemisch
(pilzliche Getreidesproßkrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22 °C und 80-90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. Bei diesem Test zeigen z.B. folgende Verbindungen eine sehr gute Wirkung, die derjenigen der aus dem Stand der Technik bekannten Verbindungen überlegen ist :

Verbindung gemäß Herstellungsbeispiel : 7.

## Herstellungsbeispiele

### Beispiel 1

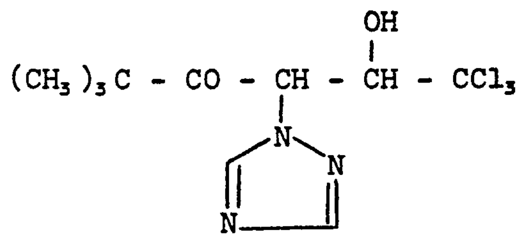

33,4 g (0,2 Mol) 3,3-Dimethyl-1-(1,2,4-triazol-1-yl)-butan-2-on werden in 150 ml Methylenchlorid gelöst und auf − 5 °C abgekühlt. Dazu tropft man langsam 19 g (0,1 Mol) Titantetrachlorid, und anschließend 29,5 g (0,2 Mol) Chloral. Während der Zudosierung soll die Innentemperatur bei − 5 °C konstant bleiben. Danach wird langsam bis zum Rückfluß erwärmt und 3 Stunden verrührt. Die Reaktionslösung wird auf Eis gegossen, und der entstehende weiße käsige Niederschlag abgesaugt. Nach Auskochen in 250 ml Methanol und anschließender Trocknung erhält man 19,9 g (32 % der Theorie) 1,1,1-Trichlor-2-hydroxy-3-(1,2,4-triazol-1-yl)-5,5-dimethyl-hexan-4-on vom Schmelzpunkt 220-222 °C.

Herstellung des Ausgangsproduktes

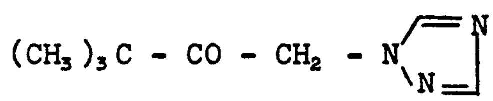

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72 % der Theorie) 3,3-Dimethyl-1-(1,2,4-triazol-1-yl)-butan-2-on vom Schmelzpunkt 62-64 °C.

### Beispiel 2

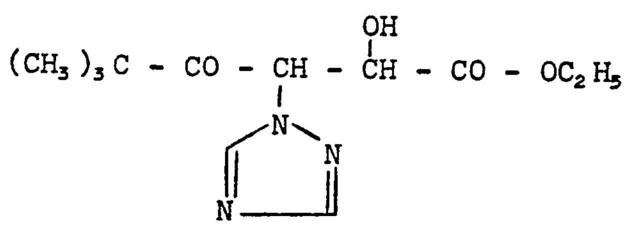

16,7 g (0,1 Mol) 3,3-Dimethyl-1-(1,2,4-triazol-1-yl)-butan-2-on werden in 400 ml Wasser gelöst. Dazu gibt man eine Lösung von 29,6 g (0,2 Mol) Glyoxylsäure-ethyl-halbacetal in 100 ml Methanol. Bei Raumtemperatur tropft man 28 ml 10 %ige Natronlauge zu. Es wird 48 Stunden nachgerührt, anschließend die Reaktionslösung dreimal mit je 200 ml Essigsäureethylester extrahiert und die vereinigten organischen Phasen über Natriumsulfat getrocknet. Nach Abdestillieren des Lösungsmittels im Vakuum erhält man ein gelbes Öl, welches langsam kristallisiert. Nach Verrühren mit 50 ml Diethylether erhält man 6,1 g (23 % der Theorie) 5,5-Dimethyl-2-hydroxy-4-oxo-3-(1,2,4-triazol-1-yl)-hexancarbonsäure-äthylester vom Schmelzpunkt 102-109 °C.

### Beispiel 3

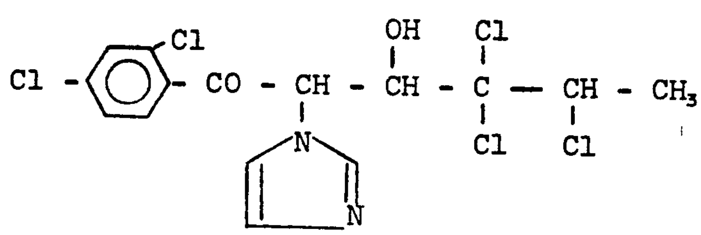

Eine Mischung aus 31,6 g (0,124 Mol) ω-Imidazol-1-yl-2,4-dichloracetophenon, 2,06 g (0,025 Mol) wasserfreiem Natriumacetat, 18 g (0,3 Mol) Eisessig und 19,3 g (0,1 Mol) 2,2,3-Trichlorbutyraldehyd wird 15 Stunden bei Raumtemperatur verrührt. Danach wird der Eisessig im Vakuum abdestilliert und der Rückstand mit 200 ml Diethylether und 200 ml Wasser verrührt. Das sich zwischen beiden Phasen abscheidende kristalline Reaktionsprodukt wird abgesaugt und getrocknet. Man erhält 32,3 g (75 % der Theorie) (2,4-Dichlorphenyl)-[(2,3,3-trichlor-4-hydroxy-5-imidazol-1-yl)-pent-5-yl]-keton vom Schmelzpunkt 138-140 °C.

Beispiel 4

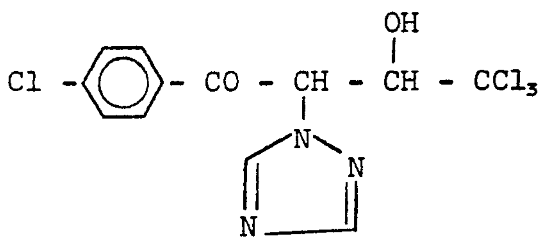

14,76 g (0,1 Mol) Chloral werden unter Rühren bei Raumtemperatur zu einer Mischung von 22,2 g (0,1 Mol) ω-(1,2,4-triazol-1-yl)-4-chloracetophenon, 5,1 g (0,063 Mol) wasserfreiem Natriumacetat und 45,4 g (0,68 Mol) Eisessig getropft. Man läßt 20 Stunden bei 100 °C nachrühren. Danach wird der Niederschlag abgesaugt, mit je 100 ml Diethylether gewaschen, mit 150 ml Methanol ausgerührt und getrocknet. Man erhält 22,1 g (60 % der Theorie) (4-Chlorphenyl)-[1,1,1-trichlor-2-hydroxy-3-(1,2,4-triazol-1-yl)-propyl]-keton vom Schmelzpunkt 173-174 °C.

In entsprechender Weise werden die nachfolgenden Beispiele der allgemeinen Formel.

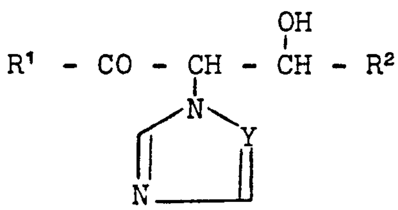

(I)

erhalten:

| Bsp. Nr. | $R^1$ | $R^2$ | Y | Schmelzpunkt (°C) |
|---|---|---|---|---|
| 5 | $(CH_3)_3C-$ | $-CO-OC_2H_5$ | N | 146-49 (reines Isomeres) |
| 6 | $(CH_3)_3C-$ | $-CCl_2-CHCl-CH_3$ | N | 198-204 |
| 7 | $ClCH_2-C(CH_3)_2-$ | $-CCl_3$ | N | 171-72 |
| 8 | $BrCH_2-C(CH_3)_2-$ | $-CCl_3$ | N | 169-71 |
| 9 | 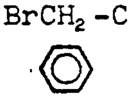 | $-CCl_3$ | N | 195-97 |
| 10 | 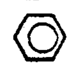 | $-CCl_2-CHCl-CH_3$ | N | 170-72 |
| 11 | 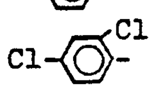 | $-CCl_3$ | N | 167-69 |
| 12 | 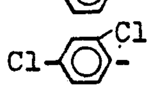 | $-CCl_2-CHCl-CH_3$ | N | 158-62 |
| 13 | 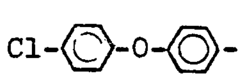 | $-CCl_3$ | N | 184-86 |
| 14 | $(CH_3)_3C-$ | $-CCl_3$ | CH | 135-38 |

| Bsp. Nr. | $R^1$ | $R^2$ | Y | Schmelzpunkt(°C) |
|---|---|---|---|---|
| 15 | $(CH_3)_3C-$ | $-CCl_2-CHCl-CH_3$ | CH | 165-66 |
| 16 | $BrCH_2-C(CH_3)_2-$ | $-CCl_3$ | CH | 132-34 |
| 17 | 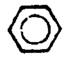 | $-CCl_3$ | CH | 151-52 |
| 18 | 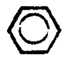 | $-CCl_2-CHCl-CH_3$ | CH | 140-41 |
| 19 | 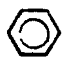 | $-CO-OC_2H_5$ | CH | 160-62 |
| 20 | 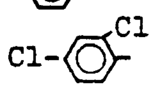 | $-CCl_3$ | CH | 141-42 |
| 21 | 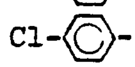 | $-CCl_3$ | CH | 145-47 |
| 22 | 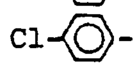 | $-CCl_2-CHCl-CH_3$ | CH | 129-31 |
| 23 | 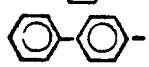 | $-CCl_3$ | CH | 145-49 |
| 24 | 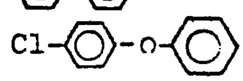 | $-CCl_3$ | CH | 147-49 |
| 25 | $BrCH_2-C(CH_3)_2-$ | $-CCl_2-CHCl-CH_3$ | CH | 138-39 |
| 26 | $(CH_3)_3C-$ | $-CCl_3$ | N | 155-56 (x $CuCl_2$) |
| 27 | $(CH_3)_3C-$ | $-CCl_3$ | CH | 108-12 (x1/2 $CuCl_2$) |
| 28 | 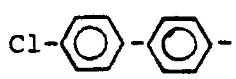 | $-CCl_3$ | CH | 233-36 |
| 29 | 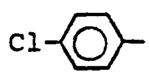 | $-CCl_3$ | CH | 118-20 (x 1/2 $CuCl_2$) |
| 30 | 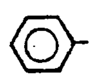 | $-CCl_2-CHCl-CH_3$ | CH | 116-20 (x 1/2 $CuCl_2$) |
| 31 | 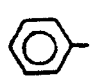 | $-CO-OC_2H_5$ | CH | 102-08 (x 1/2 $CuCl_2$) |
| 32 | 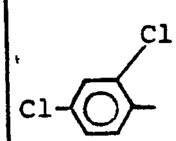 | $-CCl_2-CHCl-CH_3$ | CH | 96-98 (x 1/2 $CuCl_2$) |
| 33 | 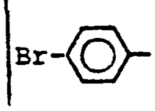 | $-CCl_3$ | CH | 151-53 |

| Bsp. Nr. | $R^1$ | $R^2$ | Y | Schmelzpunkt (°C) |
|---|---|---|---|---|
| 34 | $ClCH_2-C(CH_3)_2-$ | $-CCl_3$ | N | 145-50 ($xCuCl_2$) |
| 35 | 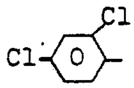 | $-CCl_2-CHCl-CH_3$ | N | 161-63 ($xCuCl_2$) |
| 36 | 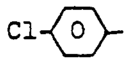 | $-CCl_3$ | N | 89-93 ($xCuCl_2$) |
| 37 | $(CH_3)_3C-$ | $-CCl_2-CHCl-CH_3$ | N | 100-05 ($xCuCl_2$) |
| 38 | $BrCH_2-C(CH_3)_2-$ | $-CCl_3$ | N | 143-45 ($xCuCl_2$) |
| 39 | 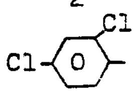 | $-CCl_3$ | N | 96-104($xCuCl_2$) |
| 40 | $FCH_2-C(CH_3)_2-$ | $-CCl_3$ | N | 121-24 ($xCuCl_2$) |
| 41 | $FCH_2-C(CH_3)_2-$ | $-CHCl_2$ | N | 121-23 ($xCuCl_2$) |
| 42 | $(CH_3)_3C-$ | $-CCl_2-CH_2Cl$ | N | |
| 43 | $ClCH_2-C(CH_3)_2-$ | $-CCl_3$ | CH | 141-43 |
| 44 | $ClCH_2-C(CH_3)_2-$ | $-CCl_2-CHCl-CH_3$ | CH | 146-47 |
| 45 | 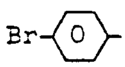 | $-CCl_2-CHCl-CH_3$ | CH | 145-47 |
| 46 | 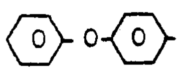 | $-CCl_3$ | CH | 161-64 |
| 47 | 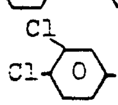 | $-CCl_3$ | CH | 155-56 |
| 48 | 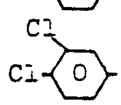 | $-CCl_2-CHCl-CH_3$ | CH | 136-37 |
| 49 | $(CH_3)_3C-$ | $-CCl_2-CHCl-CH_3$ | N | 204-10 |
| 50 | $(CH_3)_3C-$ | $-CF_3$ | N | 179-80 |
| 51 | $(CH_3)_3C-$ | $-CCl(CH_3)_2$ | N | 165-66 |
| 52 | $(CH_3)_3C-$ | $-CHCl_2$ | N | 188-90 |
| 53 | $(CH_3)_3C-$ | $-CO-OCH_3$ | N | 150-55 |
| 54 | $(CH_3)_3C-$ | $-CO-OC_4H_9$ | N | 110-14 (Reines Isomeres) |
| 55 | $(CH_3)_3C-$ | $-CO-OC_4H_9$ | N | 107-08 |
| 56 | $(CH_3)_3C-$ | $-CHCl_2$ | N | 164-65 (Reines Isomeres) |

| Bsp. Nr. | $R^1$ | $R^2$ | Y | Schmelzpunkt (°C) |
|---|---|---|---|---|
| 57 | $ClCH_2-C(CH_3)_2-$ | $-CHCl_2$ | N | 172-74 |
| 58 | $ClCH_2-C(CH_3)_2-$ | $-CCl_2-CHCl-CH_3$ | N | 169-73 |
| 59 | $FCH_2-C(CH_3)_2-$ | $-CCl_3$ | N | 195-96 |
| 60 | $FCH_2-C(CH_3)_2-$ | $-CHCl_2$ | N | 173-74 |
| 61 | 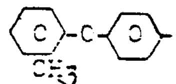 | $-CCl_3$ | N | 198-203 |
| 62 | 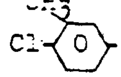 | $-CCl_3$ | N | 160-63 |
| 63 | 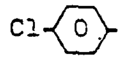 | $-CCl_2-CH_2Cl$ | N | |

**Ansprüche**

1. α-Azolyl-β-hydroxy-ketone der allgemeinen Formel

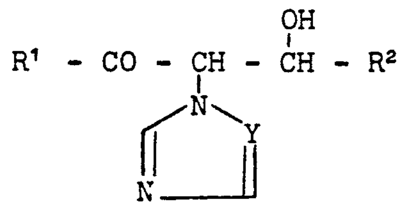

(I)

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, Alkylsulfonyloxy mit 1 bis 4 Kohlenstoffatomen und durch Phensulfonyloxy, wobei letzteres durch Alkyl mit 1 bis 2 Kohlenstoffatomen substituiert sein kann, ferner für Phenyl steht, welches gegebenenfalls substituiert sein kann durch Halogen, Alkyl mit bis zu 4 Kohlenstoffatomen, Phenyl und Phenoxy, wobei die beiden letztgenannten Reste gegebenenfalls halogensubstituiert sein können,

$R^2$ für die Gruppierung $-CX^1X^2R^3$ oder Alkoxycarbonyl mit 1 bis 4 Kohlenstoffatomen im Alkoxyteil steht, wobei,

$R^3$ für Halogen und Halogenalkyl mit 1 bis 4 Kohlenstoffatomen und 1 bis 3 Halogenatomen steht,

$X^1$ und $X^2$ gleich oder verschieden sind und für Wasserstoff oder Halogen stehen und

Y für ein Stickstoffatom oder die CH-Gruppe steht,

und deren physiologisch verträglichen Säureadditions-Salze und Metallsalz-Komplexe.

2. Verfahren zur Herstellung von α-Azolyl-β-hydroxy-ketonen der allgemeinen Formel

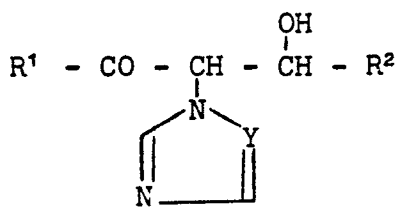

in welcher

$R^1$ für Alkyl mit 1 bis 4 Kohlenstoffatomen steht, welches gegebenenfalls substituiert sein kann durch Halogen, Alkylcarbonyloxy mit 1 bis 4 Kohlenstoffatomen im Alkylteil, Alkylsulfonyloxy mit 1 bis 4 Kohlenstoffatomen und durch Phensulfonyloxy, wobei letzteres durch Alkyl mit 1 bis 2 Kohlenstoffatomen substituiert sein kann, ferner für Phenyl steht, welches gegebenenfalls substituiert sein kann durch Halogen, Alkyl mit bis zu 4 Kohlenstoffatomen, Phenyl und Phenoxy, wobei die beiden letztgenannten Reste gegebenenfalls halogensubstituiert sein können,

$R^2$ für die Gruppierung $-CX^1X^2R^3$ oder Alkoxycarbonyl mit 1 bis 4 Kohlenstoffatomen im Alkoxyteil steht, wobei

$R^3$ für Halogen und Halogenalkyl mit 1 bis 4 Kohlenstoffatomen und 1 bis 3 Halogenatomen steht,

$X^1$ und $X^2$ gleich oder verschieden sind und für Wasserstoff oder Halogen stehen und

Y für ein Stickstoffatom oder die CH-Gruppe steht,

und deren physiologisch verträglichen Säureadditions-Salze und Metallsalz-Komplexe, dadurch gekennzeichnet, daß man die α-Azolyl-ketone der Formel

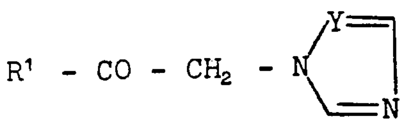

(II)

in welcher

$R^1$ und Y die oben angegebene Bedeutung haben,

mit Aldehyden der Formel

$R^2$-CHO (III)

in welcher

$R^2$ die oben angegebcne Bedeutung hat,

in Gegenwart eines Lösungsmittels und in Gegenwart eines Katalysators umsetzt, und gegebenenfalls noch die erhaltenen α-Azolyl-β-hydroxy-ketone durch Umsetzen mit Säuren in die entsprechenden Salze bzw. durch Reaktion mit Metallsalzen in die entsprechenden Metallsalz-Komplexe überführt.

3. Fungizide Mittel, gekennzeichnet durch einen Gehalt an mindestens einem α-Azolyl-β-hydroxyketon gemäß Ansprüchen 1 und 2.

4. Verwendung von α-Azolyl-β-hydroxy-ketonen gemäß Ansprüchen 1 und 2 zur Bekämpfung von Pilzen.

**Claims**

1. α-Azolyl-β-hydroxy-ketones of the general formula

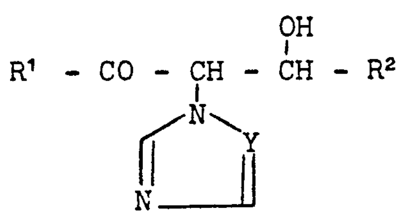

(I)

in which

$R^1$ represents alkyl with 1 to 4 carbon atoms, which can optionally be substituted by halogen, alkylcarbonyloxy with 1 to 4 carbon atoms in the alkyl part, alkylsulphonyloxy with 1 to 4 carbon atoms and by phensulphonyloxy, it being possible for the latter to be substituted by alkyl with 1 to 2 carbon atoms, it further represents phenyl, which can optionally be substituted by halogen, alkyl with up to 4 carbon atoms, phenyl and phenoxy, it being possible for the two last-mentioned radicals to be optionally substituted by halogen,

$R^2$ represents the grouping $-CX^1X^2R^3$ or alkoxycarbonyl with 1 to 4 carbon atoms in the alkoxy part, wherein

$R^3$ represents halogen and halogenoalkyl with 1 to 4 carbon atoms and 1 to 3 halogen atoms,

$X^1$ and $X^2$ are identical or different and represent hydrogen or halogen and

Y represents a nitrogen atom or the CH group, and physiologically acceptable acid addition salts and metal salt complexes thereof.

2. Process for the preparation of α-azolyl-β-hydroxy-ketones of the general formula

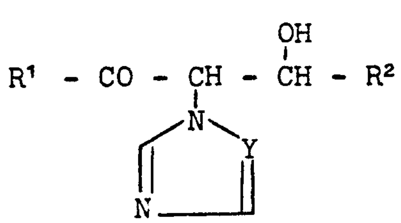

in which

$R^1$ represents alkyl with 1 to 4 carbon atoms, which can optionally be substituted by halogen, alkylcarbonyloxy with 1 to 4 carbon atoms in the alkyl part, alkylsulphonyloxy with 1 to 4 carbon atoms and by phensulphonyloxy, it being possible for the latter to be substituted by alkyl with 1 to 2 carbon atoms, it further represents phenyl which can optionally be substituted by halogen, alkyl with up to 4 carbon atoms, phenyl and phenoxy, it being possible for the two last-mentioned radicals to be optionally substituted by halogen,

$R^2$ represents the grouping $-CX^1X^2R^3$ or alkoxycarbonyl with 1 to 4 carbon atoms in the alkoxy part, wherein

$R^3$ represents halogen and halogenoalkyl with 1 to 4 carbon atoms and 1 to 3 halogen atoms,

$X^1$ and $X^2$ are identical or different and represent hydrogen or halogen and

Y represents a nitrogen atom or the CH group, and physiologically acceptable acid addition salts and metal salt complexes thereof, characterised in that the α-azolyl-ketones of the formula

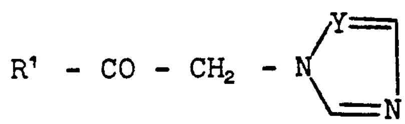

(II)

in which

$R^1$ and Y have the meaning indicated above, are reacted with aldehydes of the formula

$R^2$-CHO (III)

in which

$R^2$ has the meaning indicated above,

in the presence of a solvent and in the presence of a catalyst, and the resulting α-azolyl-β-hydroxy-ketones are optionally converted into the corresponding salts by reaction with acids or into the corresponding metal salt complexes by reaction with metal salts.

3. Fungicidal agents, characterised in that they contain at least one α-azolyl-β-hydroxy-ketone according to Claims 1 and 2.

4. Use of α-azolyl-β-hydroxy-ketones according to Claims 1 and 2 for combating fungi.

**Revendications**

1. α-azolyl-β-hydroxy-cétones de formule générale

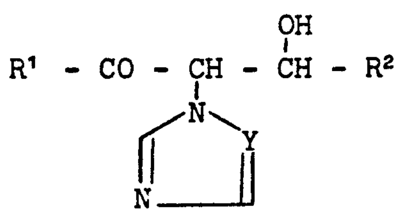

(I)

dans laquelle

$R^1$ représente un groupe alkyle contenant de 1 à 4 atomes de carbone et qui peut le cas échéant être substitué par des halogènes, des groupes alkylcarbonyloxy contenant de 1 à 4 atomes de carbone dans la partie alkyle, alkylsulfonyloxy contenant de 1 à 4 atomes de carbone et phénylsulfonyloxy, ce dernier pouvant lui-même être substitué par un groupe alkyle contenant de 1 à 2 atomes de carbone, ou un groupe phényle qui peut éventuellement être substitué par des halogènes, des groupes alkyle contenant jusqu'à 4 atomes de carbone, phényle et phénoxy, ces deux derniers restes pouvant le cas échéant être substitués par des halogènes,

$R^2$ représente le groupement $-CX^1X^2R^3$ ou un groupe alcoxycarbonyle contenant de 1 à 4 atomes de carbone dans la partie alcoxy,

$R^3$ représente un halogène ou un groupe halogénoalkyle contenant de 1 à 4 atomes de carbone et de 1 à 3 atomes d'halogènes,

$X^1$ et $X^2$, identiques ou différents, représentent l'hydrogène ou un halogène, et

Y représente un atome d'azote ou le groupe –CH,

et leurs sels d'addition d'acides et leurs complexes de sels métalliques physiologiquement acceptables.

2. Procédé de préparation des α-azolyl-β-hydroxy-cétones de formule générale

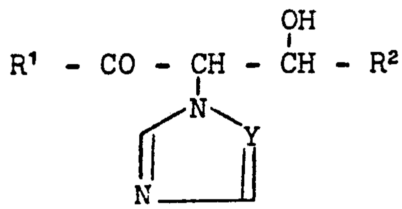

dans laquelle

$R^1$ représente un groupe alkyle contenant de 1 à 4 atomes de carbone et qui peut éventuellement être substitué par des halogènes, des groupes alkylcarbonyloxy contenant de 1 à 4 atomes de carbone dans la partie alkyle, alkylsulfonyloxy contenant de 1 à 4 atomes de carbone et phénylsulfonyloxy, ce dernier pouvant lui-même être substitué par un groupe alkyle contenant 1 ou 2 atomes de carbone, ou un groupe phényle qui peut éventuellement être substitué par des halogènes, des groupes alkyle contenant jusqu'à 4 atomes de carbone, phényle et phénoxy, ces deux derniers restes pouvant le cas échéant être substitués par des halogènes,

$R^2$ représente le groupement $-CX^1X^2R^3$ ou un groupe alcoxycarbonyle contenant de 1 à 4 atomes de carbone dans la partie alcoxy,

$R^3$ représente un halogène ou un groupe halogénoalkyle contenant de 1 à 4 atomes de carbone et de 1 à 3 atomes d'halogènes,

$X^1$ et $X^2$, identiques ou différents, représentent l'hydrogène ou un halogène et

Y représente un atome d'azote ou le groupe $-CH$,

et de leurs sels d'addition d'acides et leurs complexes de sels métalliques physiologiquement acceptables caractérisé en ce que l'on fait réagir des α-azolyl-cétones de formule

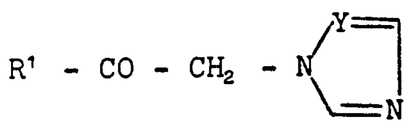

(II)

dans laquelle $R^1$ et Y ont les significations indiquées ci-dessus, avec des aldéhydes de formule

$R^2-CHO$ (III)

dans laquelle $R^2$ a la signification indiquée ci-dessus,

en présence d'un solvant et en présence d'un catalyseur, et le cas échéant on convertit les α-azolyl-β-hydroxy-cétones obtenues en les sels correspondants par réaction avec les acides ou en les complexes de sels métalliques correspondants par réaction avec des sels métalliques.

3. Produits fongicides caractérisés en ce qu'ils contiennent au moins une α-azolyl-β-hydroxy-cétone selon les revendications 1 et 2.

4. Utilisation des α-azolyl-β-hydroxy-cétones selon les revendications 1 et 2 pour combattre les mycètes.